# EUROPÄISCHE PATENTSCHRIFT

(11) **EP 2 800 595 B1**
(45) Veröffentlichungstag und Bekanntmachung des Hinweises auf die Patenterteilung: **28.06.2017**
(21) Anmeldenummer: 12797775.9
(22) Anmeldetag: 15.11.2012
(51) Int. Cl.: A61M 5/30, A61M 5/24

(54) **ZYLINDER-KOLBEN-EINHEIT MIT KLEBESCHEIBE**
BARREL-PLUNGER UNIT WITH ADHESIVE DISK
UNITÉ CYLINDRE-PISTON COMPORTANT UN DISQUE ADHÉSIF

(30) Priorität: 16.11.2011 DE 102011119058
(43) Veröffentlichungstag der Anmeldung: 12.11.2014
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Simandi, Claus
(86) Internationale Anmeldenummer: PCT/EP2012/072737
(87) Internationale Veröffentlichungsnummer: WO 2013/072418

(56) Entgegenhaltungen:
- WO-A1-2007/071485
- WO-A1-2007/088112
- WO-A2-2005/058393

## Beschreibung

Die Erfindung betrifft eine Zylinder-Kolben-Einheit, mit mindestens einem, eine Injektionslösung aufnehmenden Zylinder, mindestens einem Kolben und einer im Bereich der freien Stirnseite des Zylinders angeordneten Klebebeschichtung.

Aus der DE 10 2005 054 600 ist eine Zylinder-Kolben-Einheit eines nadelfreien Injektors bekannt, deren Zylinder an seiner vorderen Stirnseite eine mittels eines Haftklebers fixierte Dichtfolie aufweist. Der Haftkleber hat gegenüber der Zylinderstirnfläche eine größere Affinität als gegenüber der Dichtfolie, so dass er nach dem Abziehen der Dichtfolie dort zurückbleibt, um dann während der Injektion die Haut des Patienten gegenüber dem Injektor zu fixieren.

Die DE 698 36 594 T3 beschreibt eine Vorrichtung zum transkutanen Platzieren einer flexiblen Kanüle an einer Medikamenteneinführungsstelle am Patienten. Dazu besitzt die Vorrichtung einen Adaptersatz und einen spannbaren, in einem Gehäuse untergebrachten, Federspeicher. Der vorn in das Gehäuse einsetzbare Adaptersatz besteht im Wesentlichen aus einer Klebeplatte, einer flexiblen Kanüle und einer Einführungsnadel. Beim Auslösen der Vorrichtung wird der Adaptersatz mittels des sich entspannenden Federspeichers auf der Haut positioniert. Hierbei wird die Kanüle mit Hilfe der Einführungsnadel unter die Haut geschoben. Nach dem Entfernen des Gehäuses und dem Herausziehen der Einführungsnadel kann die Infusion beginnen. Die WO 2007/071485 A1 schlägt lediglich eine Klebebeschichtung der Klebescheibe vor. Bei nadelfreien Injektionen muss die Injektionslösung per Hochgeschwindigkeitsstrahl durch die Haut des Patienten appliziert werden. Die obere Hautschicht stellt eine widerstandsfähige mechanische Abschirmung der darunter liegenden Hautschichten gegenüber äußeren Einwirkungen dar. Sie besteht aus neben- und übereinander liegenden verhornten Zellen, deren Zwischenräume durch Lipide ausgekleidet sind.

Der vorliegenden Erfindung liegt daher die Problemstellung zugrunde, im Rahmen eines modular aufgebauten Injektors eine Zylinder-Kolben-Einheit zu entwickeln, deren Ausblassystem geeignet ist, die unter dem Begriff "Dermis" zusammengefassten äußeren Hautschichten oder je nach Aufgabe auch die restlichen sicher zu durchdringen, um die Injektionslösung in die Haut oder unter sie zu bringen.

Diese Problemstellung wird mit den Merkmalen des Hauptanspruchs erfindungsgemäß gelöst. Dazu weist der Zylinder einen Bodenabschnitt auf, an dem ein Ausblasrohr angeordnet ist. Am Ausblasrohr und/oder am Bodenabschnitt ist eine in Richtung der Mittellinie der Zylinder-Kolben-Einheit zwischen einer Einbauposition und einer Applikationsposition verschiebbare, elastische Klebescheibe angeordnet. Die Klebescheibe weist auf jeder der beiden Stirnseiten jeweils eine Klebebeschichtung auf. In der Klebescheibe ist in der Einbauposition das Ausblasrohr abdichtbar. Die Klebescheibe liegt in der Applikationsposition an der vorderen Stirnseite des Bodenabschnitts verklebt an. In der Applikationsposition liegt die vordere Kante der Ausblasdüse in der Ebene der vorderen Klebebeschichtung oder sie steht über diese Ebene mindestens 0,5 mm über.

Mit der Erfindung wird hier beispielsweise die Zylinder-Kolben-Einheit eines nadelfreien Injektors vorgestellt. Der Injektor, der auch ein Einmalinjektor sein kann, lagert neben der Zylinder-Kolben-Einheit einen in einem Injektorgehäuse eingebauten, auf einen Kolbenbetätigungsstempel wirkenden Antrieb. Als mögliche Antriebe können Federspeicher, Gasantriebe mit öffenbaren Gaskartuschen oder pyrotechnische Antriebe verwendet werden. Bekannte Federenergiespeicher nutzen vorgespannte mechanische oder pneumatische Federn oder Federsysteme. Wird als Antrieb ein Federenergiespeicher benutzt, wird zum Vorspannen und Halten dieses Federenergiespeichers der Kolbenbetätigungsstempel über mindestens einen am oder im Injektorgehäuse angeordneten Stützstab oder Zughaken formschlüssig gehalten. Der oder die Stützstäbe bzw. Zughaken werden mittels eines oder mehrerer Auslöseelemente bis zum Gebrauch des Injektors in ihrer Sperrposition arretiert. Zum Auslösen des Injektors werden der oder die Stützstäbe bzw. Zughaken freigegeben, so dass sich der Kolbenbetätigungsstempel - unter der Wirkung des Federenergiespeichers - zumindest annähernd parallel zur Mittellinie des Injektors bewegen kann, um die im Zylinder der Zylinder-Kolben-Einheit vorhandene Injektionslösung über mindestens eine Düse auszustoßen.

Im vorliegenden Fall ist der Zylinder-Kolben-Einheit eine am Zylinder verschiebbare Klebescheibe vorgelagert. Beim Aufsetzen des einsatzbereiten Injektors auf die Haut des Patienten verklebt in einem ersten Schritt die Haut des Patienten mit der am Zylinder in einer Einbauposition sitzenden Klebescheibe. Durch ein weiteres Andrücken des Injektors an die Haut verrutscht die Klebescheibe ggf. unter einem Auf- oder Durchstoßen der Klebescheibe.

Zugleich bedingt das Verschieben der Klebescheibe eine bereichsweise Dehnung der Haut im Injektionsbereich. Das Spannen der oberen Hautschicht im Bereich der Austrittsdüse führt je nach Hubweg der Klebescheibe zu einem ein- oder zweistufigen Auseinanderziehen der verhornten Zellen der Hornschicht der Haut. Die erste, ggf. einzige Stufe besteht im Aufweiten der zentralen Bohrung der elastischen Klebescheibe und damit dem Spannen der die Bohrung abdeckenden Haut. Diese erste Stufe endet, wenn das vordere Ende der Düse des Ausblasrohres auf der Höhe der vorderen Stirnseite der Klebescheibe zum Stillstand kommt.

Die zweite, ggf. zusätzliche Stufe besteht in einem Eindellen der Haut durch ein weiteres Verschieben der Klebescheibe im zehntel Millimeterbereich. Das Eindellen ergibt sich durch das Überstehen des vorderen Endes des Ausblasrohres über die Klebescheibe. Das Eindellen bewirkt somit eine zusätzliche Dehnung der Haut. Durch beide Stufen wird das Eindringen des Injektionsstrahls bei der Applikation erleichtert.

Weitere Einzelheiten der Erfindung ergeben sich aus den Unteransprüchen und den nachfolgenden Beschreibungen schematisch dargestellter Ausführungsbeispiele.
- Figur 1:: Zylinder-Kolben-Einheit mit angeformtem Ausblasrohr und Schutzgehäuse;
- Figur 2:: wie Figur 1, jedoch mit metallischem Ausblasrohr;
- Figur 3:: Schnitt durch den vorderen Bereich der Zylinder-Kolben-Einheit mit angeformtem, kurzen Ausblasrohr nach der Abgabe der Injektionslösung;
- Figur 4:: wie Figur 3, jedoch mit langem Ausblasrohr;
- Figur 5:: wie Figur 4, jedoch mit langem, metallischem Ausblasrohr;
- Figur 6:: Schnitt des metallischen Ausblasrohrs nach dem Aufsetzen auf die Patientenhaut vor dem vollständigen Durchstoßen der Klebescheibe;
- Figur 7:: wie Figur 6, jedoch nach dem vollständigen Durchstoßen der Klebescheibe und dem Spannen der Patientenhaut.

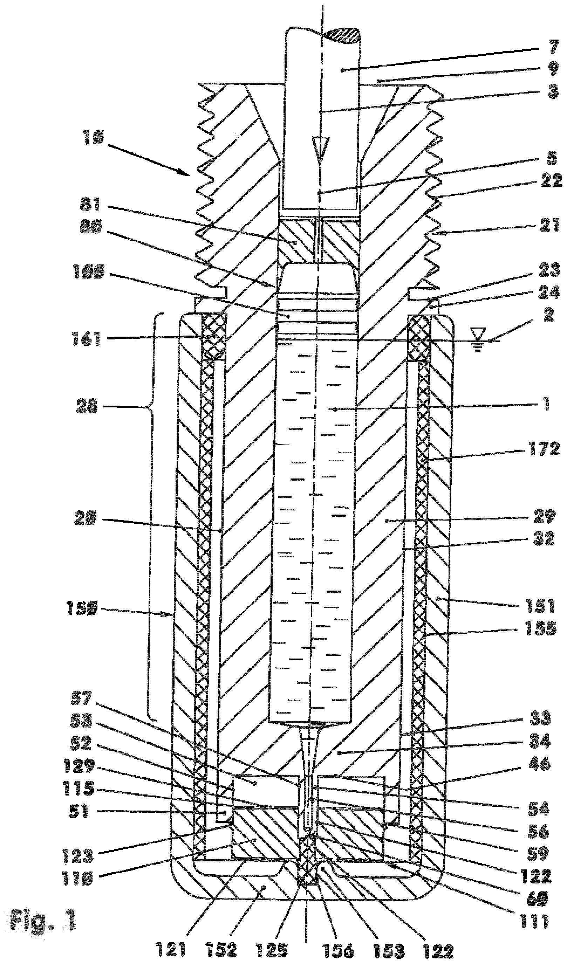

Die Figur 1 zeigt eine Zylinder-Kolben-Einheit (10) eines nadelfreien Injektors. Die Zylinder-Kolben-Einheit (10) besteht aus einem Zylinder (20) und einem z.B. zweiteiligen Kolben (80). Der Zylinder (20) ist beispielsweise zusätzlich von einem Schutzgehäuse (150) umgeben. Oberhalb des Kolbens (80) wird der untere Teil eines Kolbenbetätigungsstempels (7) gezeigt, der zu dem hier nicht dargestellten Injektor gehört. Der Zylinder (20) ist mittels seines im hinteren Bereich vorhandenen Außengewindes (22) oder mittels Schlitzen (23) am Injektor befestigt. Zwischen dem Zylinder (20) und dem Schutzgehäuse (150) ist zudem im Zylinderbodenbereich eine doppelseitig klebende Klebescheibe (110) angeordnet.

Der z.B. einteilige Zylinder (20) besteht aus einem Gehäuseadapter (21), einem Rohrabschnitt (28) und einem Bodenabschnitt (33). Mit dem Gehäuseadapter (21) wird der Zylinder (20) in einem - nicht dargestellten - Injektorgehäuse fixiert. Hierzu weist seine radiale Außenwandung ein Außengewinde (22) und/oder mindestens zwei einander gegenüberliegende Schlitze (23) auf. Die Schlitze (23) haben z.B. eine Tiefe von 2 mm. Sie befinden sich am Gewindeende in unmittelbarer Nähe des Rohrabschnitts (28). Die Breite der Schlitze (23) beträgt z.B. 0,6 mm.

Zwischen den Schlitzen (23) und dem Rohrabschnitt (28) befindet sich ein Anschlagsteg (24), dessen Außendurchmesser z.B. identisch mit dem Gewindeaußendurchmesser sein kann. Der Außendurchmesser des Rohrabschnittes (28) ist mehr als doppelt so groß wie der Durchmesser der Innenwandung (31). Er ist so dimensioniert, dass sein Werkstoff mindestens einer Druckbelastung von 350 *10⁵ Pa standhält.

An den Gehäuseadapter (21) schließt sich die Zylinderwandung (29) des Rohrabschnitts (28) an. Die Zylinderwandung (29) hat entlang der Rohrabschnittslänge z.B. eine konstante Wandstärke von 3,25 mm.

Der Bodenabschnitt (33) umfasst eine nach außen hin plane Bodenplatte (34), die der mittleren Wandstärke der Zylinderwandung (29) im Bereich des Rohrabschnitts (28) entspricht. Im äußeren Bereich der Bodenplatte (34) ist ein z.B. zylinderrohrförmiger Ringsteg (51) angeformt. Der Ringsteg (51), der einen Klebescheibenaufnahmeraum (53) umspannt, ist beispielsweise so hoch wie die Wandstärke der Bodenplatte (34). Die Wandstärke des Ringstegs (51) beträgt ca. ein Drittel der Wandstärke der Zylinderwandung (29) des Rohrabschnitts (28).

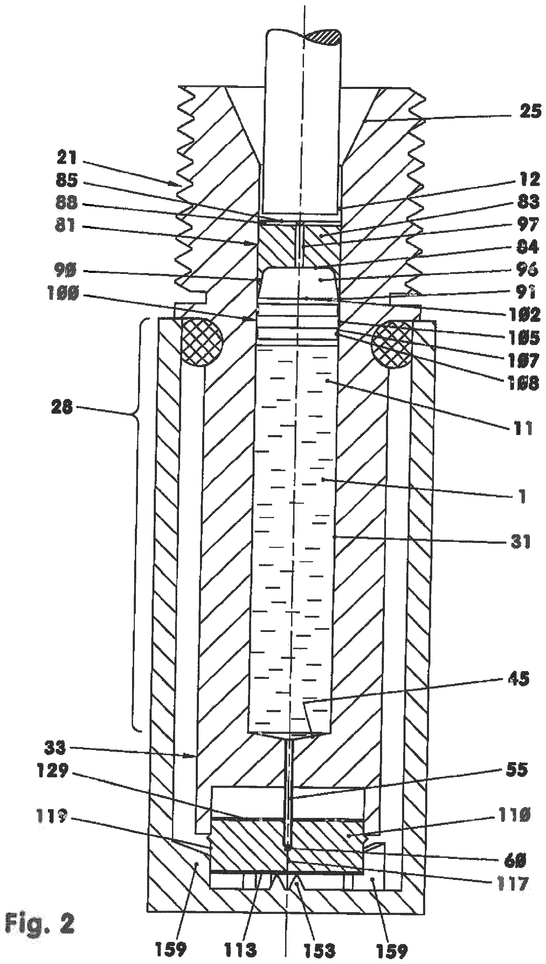

Im Zentrum der planen Bodenplatte (34) ist das die Austrittsdüse (60) tragende Ausblasrohr (54) angeordnet. Das Ausblasrohr (54), dessen Außendurchmesser z.B. 2,25 mm beträgt, hat ein vorderes Ende, das ca. einen Millimeter über den Ringsteg (51) übersteht. Zwischen der zumindest annähernd zylindrischen Außenwandung des Ausblasrohres (54) und der zylindrischen Innenwandung (52) des Ringstegs (51) liegt ein z.B. 3 mm tiefer Klebescheibenaufnahmeraum (53). Anstelle des angeformten Ausblasrohres (54) kann auch ein dünnwandiges Rohr (55) verwendet werden, vgl. Figur 2, an dessen vorderem Ende eine Austrittsdüse (60) angeordnet ist. Das dünnwandige Rohr (55), das z.B. aus einem nichtrostenden Stahl hergestellt wird, hat z.B. einen Außendurchmesser von 0,5 mm. Die Wandstärke des Ausblasrohres (55) beträgt hinter der Austrittsdüse (60) z.B. 0,05 mm. Der Düsen- bzw. der Innendurchmesser des Ausblasrohrs (55) entspricht in der Regel den aus der vorherigen Variante bekannten Werten. Nach den Figuren 2, 5, 6 und 7 misst der Innendurchmesser z.B. 0,2 mm, während der Außendurchmesser 0,36 mm beträgt. Um diese Austrittsdüse (60) herzustellen, wird der vordere Bereich des Ausblasrohres (55) auf einer Länge von ca. 0,325 mm durch Materialstauchen reduziert, so dass unmittelbar hinter der Austrittsdüse (60) eine Verjüngung (63) entsteht.

Das freie Düsenende ist z.B. mit einem Radius von 0,05 mm abgerundet, um bei der Anwendung die Haut (200) nicht zu schädigen.

Zwischen dem Ausblasrohr (54) und dem Ringsteg (51) ist nach Figur 1 im vorderen Bereich des Klebescheibenaufnahmeraums (53) eine Klebescheibe (110) angeordnet.

Sie hat eine Materialstärke, die mindestens 0,5 mm größer ist als die Tiefe des Klebescheibenaufnahmeraums (53). Die Klebescheibe (110) hat eine zentrale Bohrung (122), deren Innendurchmesser z.B. um 0,5 - 1 mm kleiner ist als der Außendurchmesser des Ausblasrohres (54). Somit wird der vordere Bereich des Ausblasrohres (54) dicht und eng anliegend vom hinteren Bereich der Bohrung (122) umgeben. Der vordere, noch ungedehnte Bereich der Bohrung (122) erscheint somit in Figur 1 mit einem kleineren Durchmesser.

Für die Zylindervariante mit dem eingeklebten oder umspritzten Ausblasrohr (55) wird eine Klebescheibe (110) verwendet, die keine Bohrung aufweist. Der nach Figur 2 vor dem Ausblasrohr (55) gelegene Bereich der Klebescheibe (110) wird als Dichtbereich (117) bezeichnet.

Die im Wesentlichen zylindrische Außenwandung der Klebescheibe (110) ist an der zylindrischen Innenwandung (52) des Ringstegs (51) geführt. Nach Figur 1 hat die Klebescheibe (110) im oberen Bereich ihrer Außenwandung einen radial, z.B. 0,5 mm, überstehenden Umlaufsteg (123), über den sie an der vorderen Innenkante (59) des Ringstegs (51) elastisch anliegt.

Zur Positionierung der Klebescheibe (110) am Ringsteg (51) des Bodenabschnitts (33), kann letzterer auch einen im vorderen Bereich des Ringstegs (51) angeformten, radial nach innen ragenden Steg aufweisen, der in eine entsprechende Ringnut der Klebescheibe (110) elastisch hineinragt.

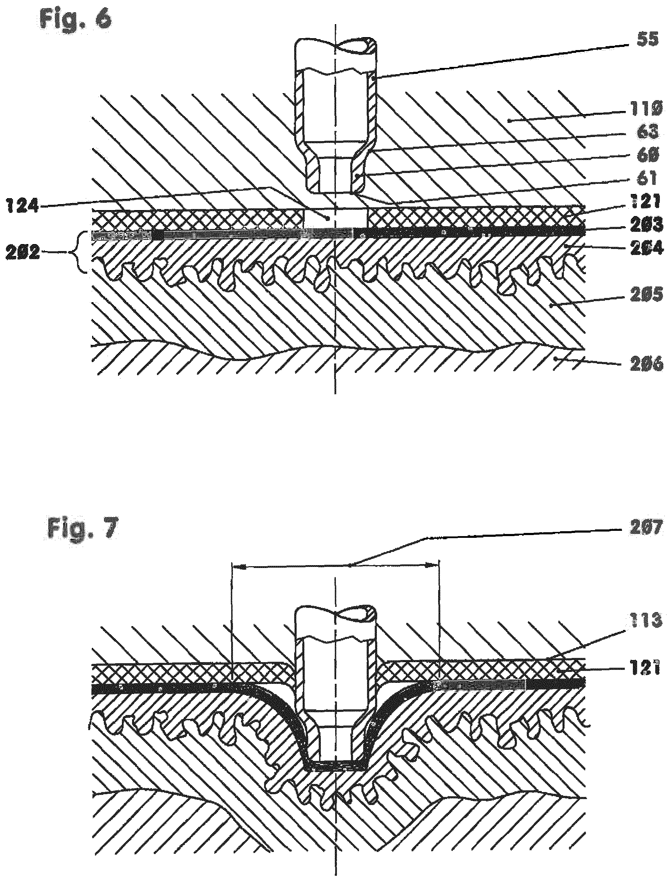

Die z.B. aus Gummi oder einem anderen Elastomer gefertigte Klebescheibe (110) ist an ihren beiden z.B. plan ausgeführten Stirnseiten jeweils mit einer z.B. aus einem Haftkleber bestehenden Klebeschicht (121, 129) ausgestattet. Die restlichen Oberflächenbereiche haben eine gute Gleitfähigkeit, da die Klebescheibe (110) zumindest partiell mit Silikonöl behandelt oder teflonisiert wird. Bei der für das Ausblasrohr (54) geeigneten Variante, vgl. Figur 1, 3 und 4, ist die Klebeschicht (121) im Bereich der Bohrung (122) ausgespart. Bei der Variante für das metallische Ausblasrohr (55) kann die Klebeschicht (121) eine zentrale Ausnehmung (124) haben, deren Durchmesser zumindest größer ist, als der Außendurchmesser des Ausblasrohres (55) im Bereich der Austrittsdüse (60), vgl. Figur 6.

Der Haftkleber der Klebescheibe (121) ist so ausgelegt, dass seine Klebekraft gegenüber der Klebescheibe (110) um mindestens 50% größer ist als gegenüber einer desinfizierten Hautoberfläche (201).

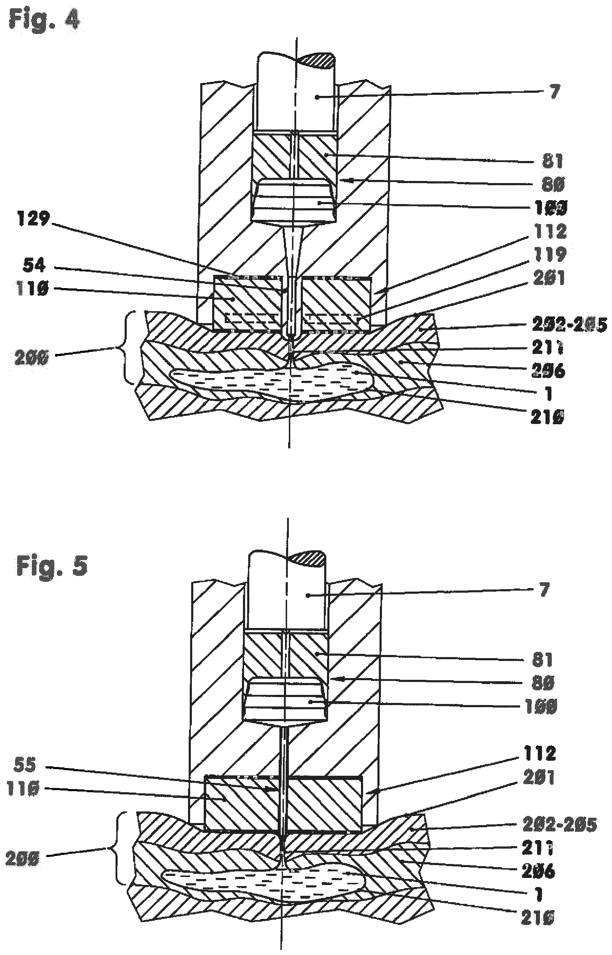

In die Klebescheibe (110) kann eine Versteifungsscheibe (119) eingesetzt werden. Sie ist in Figur 4 gestrichelt dargestellt. Diese z.B. umspritzte oder einvulkanisierte Versteifungsscheibe (119) hat eine Wandstärke von z.B. 0,5 - 1 mm. Sie ist beispielsweise aus einem üblichen Eisen- oder Buntmetall gefertigt. Ihre Bohrung ist mindestens 1 mm größer als der Außendurchmesser des Ausblasrohres (54). Der Außendurchmesser der Versteifungsscheibe (119) ist z.B. 1 - 2 mm kleiner als der Außendurchmesser der Klebescheibe (110). Die hier in die Klebescheibe (110) integrierte Versteifungsscheibe (119) ist z.B. 0,5 bis 1 mm hinter der vorderen Klebeschicht 121) positioniert. Die Mittellinien der Klebescheibe (110) und der Versteifungsscheibe (119) sind deckungsgleich.

Ggf. hat die Klebescheibe (110) seitlich mindestens eine parallel zur Mittellinie (5) orientierte Kerbe, die es beim Einschieben der Klebescheibe (110) in den Klebescheibenaufnahmeraum (53) ermöglicht, die dort vorhandene Luft problemlos zu verdrängen. Die Luft kann auch über eine im Ringsteg (51) in der Nähe der Stirnfläche (46) des Bodenabschnitts (33) angeordnete Bohrung entweichen.

Das topfförmige Schutzgehäuse (150) nach Figur 1, ein Sterilverschluss, der beispielsweise aus Glas gefertigt ist, besteht hier aus einem rohrförmigen Mantel (151) und einem planen Boden (152). Die zylindrische, glatte Außenwandung (32) des Rohrabschnitts (28) und des Bodenabschnitts (33), mit der eingesetzten Klebescheibe (110) des Zylinders (20), wird hierbei von dem Schutzgehäuse (150) umgeben. Im Bereich des Rohrabschnitts (28) beträgt der Abstand zwischen dessen Außenwandung (32) und der Innenwandung (155) des Schutzgehäuses (150) z.B. 1,5 mm. Der axiale Abstand zwischen dem Boden (152) des Schutzgehäuses (150) und der Klebescheibe (110) beträgt nach Figur 1 z.B. 1 mm.

Am Zylinder (20) ist das Schutzgehäuse (150) an zwei Stellen lösbar fixiert. Die erste Stelle liegt am Übergang zwischen dem Rohrabschnitt (28) und dem Anschlagsteg (24) des Zylinders (20). Dort befindet sich, nach Figur 1, ein O-Ring (161), über den das Schutzgehäuse (150) gegenüber dem Zylinder (20) abgedichtet ist. Zugleich zentriert der O-Ring (161) das Schutzgehäuse (150) am Zylinder (20). Anstelle eines konventionellen O-Rings (161) kann auch ein Quadring, ein Profilring oder dergleichen verwendet werden.

Der Dichtring (161) wird bei der Montage zwischen dem Schutzgehäuse (150) und dem Zylinder (20) verklemmt, so dass er neben der Dichtfunktion auch problemlos eine Haltefunktion übernehmen kann. Ggf. kann der Dichtring (161) auch durch einen abdichtenden, zähbleibenden Klebstoff ersetzt werden.

Die zweite Stelle zur Abstützung des Schutzgehäuses (150) am Zylinder (20) befindet sich mittig im Boden (152) des Schutzgehäuses (150). Dort ist eine zentrale Sacklochbohrung (156) angeordnet, die von einem am Boden (152) angeformten, nach innen vorstehenden Stützsteg (153) umgeben wird. Der ringförmige Stützsteg (153) liegt mit seiner z.B. halbtorusförmigen Stirnseite an der Klebescheibe (110) an.

In der Sacklochbohrung (156) steckt eingeklemmt oder eingeklebt ein abgestufter Gummistopfen (125). Letzterer sitzt abdichtend mit seinem hinteren Ende vor der Austrittsdüse (60) des Ausblasrohres (54). Sein vorderes, in der Sacklochbohrung (156) steckendes Ende weist einen Durchmesser auf, der z.B. 0,5 mm größer ist als der seines hinteren Endes. Der Gummistopfen (125) fixiert in radialer Richtung das vordere Ende des Schutzgehäuses (150) über die Klebescheibe (110), die sich am Ringsteg (51) des Zylinders (20) abstützt.

Im Ausführungsbeispiel nach Figur 2 ist das Schutzgehäuse (150) aufgrund seiner Formgebung z.B. aus dem Kunststoff Cycloolefin Copolymer (COC) gefertigt. Dieser Werkstoff hat eine besonders niedrige Gas- und Wasserdampfdurchlässigkeit.

Um bei der Variante das Schutzgehäuse (150) im vorderen Bereich am Zylinder (20) auch in Radialrichtung abstützen zu können, weist das Schutzgehäuse (150) z.B. fünf radial orientierte Stützrippen (159) auf. Diese am Umfang des Mantels (151) äquidistant verteilten Stützrippen (159) sind beispielsweise am Boden (152) und am Mantel (151) angeformt. Die Stützrippen (159) haben radiale Innenflächen, mit denen sie an der zylindrischen Außenfläche (119) der Klebescheibe (110) anliegen.

Zur axialen Stützung der Klebescheibe (110) ist zudem am Boden (152) ein ringförmiger Stützsteg (153) angeordnet, der mit seiner oberen, kreisförmigen Kante an der Klebescheibe (110), in deren Randbereich, anliegt. Die Kante ist hierbei so schmal, dass sie gegenüber der Klebescheibe (110) nur eine geringe Haftkraft entwickelt.

Nach den Figuren 1 und 2 ist der Zylinder (20) mit einer Injektionslösung (1) zumindest teilbefüllt. Der Flüssigkeitsspiegel (2) der Injektionslösung (1) befindet sich im Übergangsbereich zwischen dem Gehäuseadapter (21) und dem Rohrabschnitt (28). Auf dem Flüssigkeitsspiegel (2) ist blasenfrei und steril ein scheibenförmiger Dichtkörper (100) aufgesetzt, der unter einer radialen Klemmwirkung dichtend an der Zylinderinnenwandung (31) anliegt. Hinter dem Dichtkörper (100) ist ein topfförmiger Treibkörper (81) angeordnet. Der Treibkörper (81) liegt dabei am Dichtkörper (100) partiell an oder er hat einen Abstand von z.B. 0,2 bis 0,5 mm.

Der Dichtkörper (100) ist hier eine Scheibe, deren unverformter Durchmesser z.B. doppelt so groß ist wie seine Scheibendicke. Am Umfang kann die Scheibe (100) ein Rillenprofil (107) aufweisen, das z.B. zwei Rillen (108) hat, vgl. Figur 2. Das Rillenprofil (107) ist hier beispielsweise so gestaltet, dass der Dichtkörper (100) im Querschnitt beidseitig als Schnittprofil eine Wellenlinie mit zwei, die Rillen (108) formenden Wellentälern aufweist. Die Wellenlinie ist hierbei aus Kreisbögen zusammengesetzt.

Da der Dichtkörper (100) ein Elastomerkörper ist, sind die Wellenberge der gesetzten Dichtscheibe abgeplattet, vgl. Figuren 1, 2, 3, 4 und 5.

Der topfförmige Treibkörper (81), dessen Länge z.B. seinem Außendurchmesser entspricht, besteht aus einer scheibenförmigen Schlagplatte (83) und einer angeformten Schürze (90). Die Dicke der Schlagplatte (83) ist hierbei geringfügig größer als die Länge der Schürze (90), vgl. Figur 2.

Die Schlagplatte (83), auf die beim Auslösen des Injektors der Kolbenbetätigungsstempel (7) aufschlägt, hat mindestens eine z.B. zentrale Bohrung (97), die die vor und hinter dem Treibkörper (81) gelegenen Zylinderraumbereiche (11, 12) miteinander mit minimaler Drosselwirkung verbindet. Die Bohrung (97), die auch schräg zur Mittellinie (5) orientiert sein kann und deren minimaler Durchmesser zwischen 1 und 2 mm liegt, endet nach den Ausführungsbeispielen an der hinteren Stirnseite (85) des Treibkörpers (81), z.B. in einem Kanalkreuz (88) aus zwei sich im Bereich der Bohrung (97) schneidenden Kanälen. Die Kanäle des Kanalkreuzes (88) haben jeweils einen halbkreisförmigen Querschnitt, wobei der Durchmesser der Querschnitte z.B. dem Durchmesser der Bohrung entspricht.

An der vorderen Stirnseite (84) der Schlagplatte (83) schließt sich die als elastische Dichtlippe ausgebildete Schürze (90) an. Die Wandung der Schürze (90) verjüngt sich, ausgehend von der Stirnseite (84), hin zur vorderen, äußeren Dichtkante (91), die elastisch an der Zylinderinnenwandung (31) in jedem Betriebszustand des Injektors anliegt. Im eingebauten Zustand umschließen die Schürze (90) und die vordere Stirnseite (84) einen Eintauchhohlraum (96). Letzterer hat im Wesentlichen die Form eines Kegelstumpfes, dessen Kegelwinkel z.B. 20 Winkelgrade misst.

Der Kolben (80), also die Kombination aus dem Treibkörper (81) und dem Dichtkörper (100), ermöglicht ein einfaches blasenfreies, steriles Befüllen und ein Verschließen der Zylinder-Kolben-Einheit (10) in Verbindung mit einem Ausstoßvorgang beim Auslösen des Injektors, der einem sehr hohen Verdichtungsstoß von bis zu 350 * 10⁵ Pa standhält.

Wird der Injektor für die Injektion vorbereitet, wird das Schutzgehäuse (150) nach vorn vom Zylinder (20), z.B. mittels Handkraft, abgezogen.

Wird der Injektor für die Injektion vorbereitet, wird das Schutzgehäuse (150) zusammen mit dem Baumwollvlies (172) nach vorn vom Zylinder (20), z.B. mittels Handkraft, abgezogen. Hierbei bleibt, bei der Variante nach Figur 1, der Gummistopfen (125) am Schutzgehäuse (150) hängen, während der Dichtring (161) an der Außenwandung (32) des Zylinders (20) zurückbleibt. Die Klebescheibe (110) verharrt im Bodenabschnitt (33) des Zylinders (20) in der aus Figur 1 bekannten Position.

Um die Applikation der Injektionslösung durchführen zu können, wird der Injektor mit der Klebescheibe (110) voraus auf die Hautoberfläche (201) des Patienten aufgesetzt. Dabei verklebt die Klebescheibe (110), die sich noch in ihrer Einbauposition (111) befindet, mit ihrer Klebeschicht (121), die z.B. bei einem Durchmesser von 10 mm eine Fläche von ca. 75 mm² hat, mit der Hautoberfläche (201).

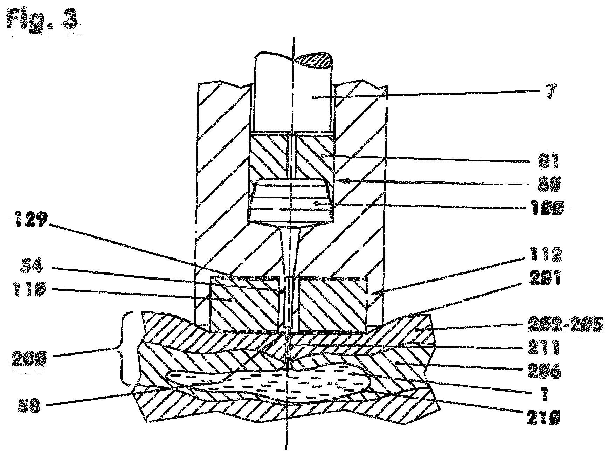

Durch die Anpresskraft des Injektors wird die Klebescheibe (110) so belastet, dass sie-unter einem Überwinden der Sperrwirkung des Umlaufstegs (123) - entlang des Ausblasrohrs (54) in Richtung des Bodenabschnitts (33) verschoben wird. Während sich hierbei die vordere Stirnfläche (58) des Ausblasrohres (54) auf die Haut zubewegt, dehnt das Ausblasrohr (54) die vordere Sacklochbohrung um ca. 0,5 mm auf. Diese Aufdehnung führt dazu, dass die mit der Klebeschicht (121) verklebte Hautschicht in einer ersten, ggf. einzigen Stufe - allein durch die Aufweitung der Sacklochbohrung (126) - aktiv gespannt wird, vgl. Figur 3. Der Dehnfaktor ist der Quotient aus dem Quadrat des Außendurchmessers des Ausblasrohres (54) und dem Quadrat des Innendurchmessers der noch ungedehnten Bohrung (122).

Für den Fall, dass die Haut (200) nur einstufig gedehnt werden soll, wird der Hubweg der Klebescheibe (110) - durch die geometrische Abstimmung der Höhe des Ringsteges (51) oder die Länge des Ausblasrohres (54) und die Wandstärke der Klebescheibe (110) - so gewählt, dass die vordere Kante (61) der Austrittsdüse (60) des Ausblasrohres (54, 55) zumindest annähernd in der Ebene liegt, die als vordere Stirnebene der vorderen Klebeschicht (121) zu betrachten ist. In Figur 3 hat das verkürzte Ausblasrohr (54) z.B. eine plane Stirnfläche (58), die speziell in dieser Ebene zum Stillstand kommt.

Da die Klebeschicht (121) in der Regel sehr dünnwandig ist, genügt es, wenn sich die Stirnfläche (58) irgendwo zwischen der hinteren und der vorderen Stirnfläche der Klebeschicht (121) befindet.

Nach Abschluss der ersten Stufe liegt die Klebescheibe (110) mit ihrer hinteren Klebeschicht (129) an der Stirnfläche (46) des Bodenabschnitts an, womit die Abgabe der Injektionslösung (1) eingeleitet werden kann.

Alternativ hierzu kann die Dehnung der Haut (200) in zwei Stufen stattfinden. Zu der im vorherigen Abschnitt beschriebenen ersten Stufe kommt eine zweite hinzu, die durch eine weitere Verschiebung der Klebescheibe (110) eingeleitet wird. In diesem Fall sind zwischen der Klebescheibe (110) und der Stirnfläche (46) noch einige zehntel Millimeter Spiel vorhanden.

Bei der weiteren Vorwärtsbewegung des Injektors rutscht die Klebescheibe (110)-unter einem Aufbrauchen des noch vorhandenen Spiels - weiter in den Klebescheibenaufnahmeraum (53) hinein, um sich mit ihrer hinteren Stirnseite (115) an der Stirnfläche (46) des Bodenabschnitts (33) anzulegen. Die Klebescheibe (110) befindet sich jetzt in ihrer Applikationsposition (112). Sie füllt nun den Klebescheibenaufnahmeraum (53) komplett aus.

Aufgrund dieses zweiten Hubs der Klebescheibe (110) wird zum einen durch die Erhöhung des Anpressdrucks die Verklebung zwischen der Klebeschicht (121) und der Haut des Patienten verstärkt und zum anderen tritt das Ausblasrohr (54) einige zehntel Millimeter aus der Klebescheibe (110) hervor, vgl. Figur 4.

Dabei drückt die vordere Stirnseite (58) des hervorstehenden Ausblasrohres (54) mit der beispielsweise nahezu sphärisch gekrümmten Stirnfläche (58) eine Vertiefung in die Haut (200), wodurch sich die zweite Stufe der Hautdehnung einstellt. Die hierbei entstehende Beulentiefe, das ist der Abstand zwischen der unteren Klebeschicht (121) und dem vordersten Punkt oder der vordersten Kante des Ausblasrohres (54), entspricht z.B. dem halben Außendurchmesser des Ausblasrohres (54). In diesem Fall wird die Hautoberfläche (201) im Bereich der Stirnfläche (58) des Ausblasrohres (54) in der Summe aktiv um ca. 100% gedehnt.

Wird der Injektor aus Figur 2 benutzt, so ergibt sich prinzipiell eine vergleichbare zweistufige Hautdehnung, wie bei dem Beispiel nach Figur 1. Aufgrund des relativ schlanken Ausblasrohres (55) mit seiner Verjüngung (63) im Bereich der Austrittsdüse (60) gibt es je nach Hauttyp zwei verschiedene Anlagebedingungen für die zu dehnende Haut (200).

Bei einer ersten Anlagebedingung schmiegt sich die Haut (200) beim Heraustreten des Ausblasrohres (55) an der Außenwandung (57) an, so dass die Oberfläche (201) der gedehnten Haut (200) die Form einer gestuften Büchse annimmt, wobei die Büchsenstufung durch die Verjüngung (63) des Ausblasrohres (55) zustande kommt. Bei der zweistufigen Hautdehnung ergibt sich bei dieser Variante z.B. ein Dehnungsfaktor von 3,5 bis 4.

Bei einer zweiten Anlagebedingung wird die Hornschicht (203) - ohne die Haut (200) zu schädigen - durch die Austrittsdüse (60) bereichsweise von der Klebeschicht (121) abgehoben bzw. gelöst. Der Abhebebereich hat z.B. einen Durchmesser (207), der bis zu 1 mm betragen kann.

Die Haut (200) wird hier unter der Entstehung einer größeren Dehnung im Bereich um die Austrittsdüse (6) eine z.B. trichterähnliche Gestalt annehmen, vgl. Figur 7. Hierbei werden die Epidermis (202) und die Dermis (205) bis in die Unterhaut (206) ausgebeult. Bei den geometrischen Verhältnissen nach Figur 7, bei der die Hornschichtbeulung ca. dem halben Durchmesser (207) der von der Klebeschicht (121) abgelösten Hautoberfläche (201) entspricht, stellt sich dort im Bereich der trichterähnlichen Beulung eine Hornschichtdehnung von 60 bis 70% ein.

Am zeitlichen Ende der ein- oder zweistufigen Hautdehnung wird der Injektor, durch die auf ihn wirkende Andruckkraft des Patienten ausgelöst. Der mittels einer mechanischen oder pneumatischen Feder vorgespannte Kolbenbetätigungsstempel (7) belastet schlagartig den Kolben (80), um mit der Injektionslösung (1) per Hochgeschwindigkeitsstrahl die gespannte Haut des Patienten zu durchschlagen.

Dabei schlägt der Kolbenbetätigungsstempel (7) zunächst mit großer Wucht gegen den Treibkörper (81), siehe Pfeilrichtung (3) in Figur 1. Der Treibkörper (81) wird gegen die nahezu inkompressibel auf dem Flüssigkeitsspiegel (2) aufliegende Dichtscheibe (100) gepresst. Hierbei schiebt sich die Schürze (90) entlang der Zylinderinnenwandung (31) über die z.B. profilierte Außenwandung (105) der Dichtscheibe (100). Die Dichtscheibe (100) taucht in den Eintauchhohlraum (96) des Treibkörpers (81) ein, vgl. hierzu Figuren 3 bis 5. Die dabei verdrängte Luft strömt über die Bohrung (97) und das Kanalkreuz (88) am Kolbenbetätigungsstempel (7) entlang in die äußere Injektorumgebung (9).

Nun bilden die Dichtscheibe (100) und der Treibkörper (81) einen quasistarren Verbund, den Kolben (80), der die Injektionslösung (1) vor sich herschiebt. Die Abdichtung gegenüber der Zylinderinnenwandung (31) übernimmt die Dichtkante (91) der Schürze (90), die durch die flüssigkeitsdruckbelastete Dichtscheibe (100) verstärkt angepresst wird. Da der Gleitreibungskoeffizient der Dichtkante (91), aufgrund des verwendeten Treibkörperwerkstoffes, kleiner ist als der Gleitreibungskoeffizient der Dichtscheibe (100), ergibt sich trotz hoher Dichtwirkung ein geringer Gleitreibungswiderstand.

Durch den hohen Flüssigkeitsdruck, er misst z.B. für die subkutane Injektion bei einer Ausströmgeschwindigkeit von ca. 150 m/sec mindestens 250 * 10⁵ Pa, durchdringt der Flüssigkeitsstrahl die Epidermis (202) und die Dermis (205), die zusammen z.B. bis zu 2 mm dick sind, vgl. Figuren 3 bis 5. Ein durch den Flüssigkeitsstrahl erzeugter Einströmkanal (211) endet bei der subkutanen Injektion erst in der Unterhaut (206). In dem von Kapillargefäßen durchzogenen fettgewebsreichen Bindegewebe der Subcutis (206) entsteht dann eine vom Flüssigkeitsstrahl gespeiste, z.B. diskusförmige, Injektionslösungsansammlung (210).

Nach der Entleerung des Zylinders (20) wird der Injektor von der Haut (200) gelöst. Da die Verklebung zwischen den Zylinder (20) und der Klebscheibe (110) grundsätzlich stärker ist als zwischen der Klebescheibe (110) und der Haut (200) erfolgt dies problem- und schmerzlos.

Die unter Druck stehende Injektionslösungsansammlung (210) wird zumindest großteils in der Haut zurückgehalten, da sich die Dehnung der Haut nach der Wegnahme des Injektors sofort abbaut und somit der Einströmkanal (211) wieder verschlossen wird.

### Bezugszeichenliste:

- 1: Injektionslösung
- 2: Flüssigkeitsspiegel
- 3: Pfeilrichtung bei Injektorauslösung
- 5: Mittellinie
- 7: Kolbenbetätigungsstempel
- 9: Umgebung

- 10: Zylinder-Kolben-Einheit
- 11: Zylinderraumbereich, vor dem Kolben
- 12: Zylinderraumbereich, hinter dem Kolben

- 20: Zylinder
- 21: Gehäuseadapter
- 22: Außengewinde
- 23: Schlitze
- 24: Anschlagsteg
- 25: Aufweitung, innen

- 28: Rohrabschnitt
- 29: Zylinderwandung
- 31: Innenwandung, radial
- 32: Außenwandung, radial
- 33: Bodenabschnitt
- 34: Bodenplatte

- 45: Zylinderboden, Innenseite des Zylinderbodens
- 46: Stirnfläche des Bodenabschnitts, vorn
- 51: Ringsteg
- 52: Innenwandung, zylindrisch
- 53: Klebescheibenaufnahmeraum
- 54: Ausblasrohr, Kunststoff
- 55: Ausblasrohr, Rohr, Stahlrohr
- 56: Bohrung, Innenbohrung
- 57: Außenwandung
- 58: Stirnseite, Stirnfläche
- 59: Kante, vordere
- 60: Düse, Austrittsdüse
- 61: Kante, vorn
- 63: Verjüngung

- 80: Kolben, Kombination aus (81) und (100)
- 81: Treibkörper
- 83: Schlagplatte
- 84: Stirnseite, vorn
- 85: Stirnseite, hinten
- 88: Kanalkreuz

- 90: Schürze, elastisch; Dichtlippe
- 91: Kante, Dichtkante
- 96: Eintauchhohlraum, Hohlraum
- 97: Ausnehmung, Bohrung, zentral

- 100: Dichtkörper, Dichtscheibe
- 102: Stirnseite, hinten
- 105: Außenwandung, profiliert
- 107: Rillenprofil
- 108: Rille

- 110: Klebescheibe, Elastomerscheibe
- 111: Einbauposition
- 112: Applikationsposition
- 113: Stirnseite, vorn; Stirnfläche
- 115: Stirnseite, hinten
- 117: Dichtbereich
- 119: Außenfläche, radial
- 121: Klebeschicht, vorn, Haftkleber, Klebebeschichtung
- 122: Bohrung, gestuft
- 123: Umlaufsteg
- 124: Ausnehmung in (121)
- 129: Klebeschicht, hinten, Haftkleber, Klebebeschichtung

- 150: Schutzgehäuse, Glas; Schale, außen; Sterilverschluss
- 151: Mantel, rohrförmig
- 152: Boden, plan
- 153: Stützsteg
- 155: Innenwandung
- 157: Stützzapfen
- 159: Stützrippen
- 161: O-Ring

- 200: Haut
- 201: Hautoberfläche
- 202: Epidermis
- 203: Hornschicht (Stratum Corneum)
- 204: Hornbildungs- und Regenerationsschicht
- 205: Papillar- und Geflechtsschicht (Dermis)
- 206: Unterhaut (Subcutis)
- 207: Durchmesser der abgelösten Hautoberfläche (201)

- 210: Injektionslösungsansammlung
- 211: Einströmkanal

## Patentansprüche

1. Zylinder-Kolben-Einheit (10), mit mindestens einem, eine Injektionslösung aufnehmenden Zylinder (20), mindestens einem Kolben (80) und einer im Bereich der freien Stirnseite des Zylinders (20) angeordneten Klebebeschichtung (121), wobei
- der Zylinder (20) einen Bodenabschnitt (33) aufweist, an dem ein Ausblasrohr (54, 55) mit einer Ausblasdüse (60) angeordnet ist,
- am Ausblasrohr (54, 55) und/oder am Bodenabschnitt (33) eine in Richtung der Mittellinie (5) der Zylinder-Kolben-Einheit (10) zwischen einer Einbauposition (111) und einer Applikationsposition (112) verschiebbare, elastische Klebescheibe (110) angeordnet ist,
- die Klebescheibe (110) auf jeder der beiden Stirnseiten (113, 115) jeweils eine Klebebeschichtung (121, 129) aufweist,
- in der Klebescheibe (110) in der Einbauposition (111) das Ausblasrohr (54, 55) abdichtbar ist,
- die Klebescheibe (110) in der Applikationsposition (112) an der vorderen Stirnseite (46) des Bodenabschnitts (33) verklebt anliegt und
- in der Applikationsposition (112) die vordere Kante (61) der Ausblasdüse (60) in der Ebene der vorderen Klebebeschichtung (121) liegt oder über diese Ebene mindestens 0,5 mm übersteht.

2. Zylinder-Kolben-Einheit gemäß Anspruch 1,
**dadurch gekennzeichnet,**
- **dass** die sich in Einbauposition (111) befindende Klebescheibe (110) mindestens einen Millimeter von der ihr zugewandten vorderen Stirnseite (46) des Bodenabschnitts (33) entfernt ist.

3. Zylinder-Kolben-Einheit gemäß Anspruch 1,
**dadurch gekennzeichnet,**
- **dass** die Klebescheibe (110) ein Elastomerkörper ist, der zumindest in Längsrichtung also parallel zur Mittellinie (5) der Zylinder-Kolben-Einheit (10) - elastisch ausgebildet ist.

4. Zylinder-Kolben-Einheit gemäß Anspruch 1,
**dadurch gekennzeichnet,**
- **dass** die Außenwandung (57) des Ausblasrohres (54, 55) zumindest bereichsweise zylindrisch geformt ist.

5. Zylinder-Kolben-Einheit gemäß Anspruch 1,
**dadurch gekennzeichnet,**
- **dass** der Bodenabschnitt (33) einen Ringsteg (51) aufweist, der sich nach vorn in einer Verlängerung der Zylinderaußenwandung (32) erstreckt.

6. Zylinder-Kolben-Einheit gemäß Anspruch 5,
**dadurch gekennzeichnet,**
- **dass** der Ringsteg (51) eine zylindrische Innenwandung (52) aufweist.

7. Zylinder-Kolben-Einheit gemäß Anspruch 1,
**dadurch gekennzeichnet,**
- **dass** die Klebescheibe (110) eine Bohrung (122) aufweist, die durch ein Dichtelement (125) verschlossen ist.

8. Zylinder-Kolben-Einheit gemäß Anspruch 1,
**dadurch gekennzeichnet,**
**dass** die Klebescheibe (110) aus Gummi oder einem Elastomer besteht oder partiell mit Silikon behandelt oder teflonisiert ist.

9. Zylinder-Kolben-Einheit gemäß Anspruch 5,
**dadurch gekennzeichnet,**
**dass** die Klebescheibe (110) einen radial überstehenden Umlaufsteg (123) aufweist, welcher an der vorderen Innenkante (59) des Ringstegs (51) anliegt.

## Claims

1. A barrel-plunger unit (10) having at least one barrel (20) that holds an injection solution, at least one plunger (80), and an adhesive coating (121) disposed in the region of the free face side of the barrel (20), wherein
- the plunger (20) has a bottom section (33) on which a blow-out pipe (54, 55) having a blow-out nozzle (60) is disposed,
- an elastic adhesive disk (110) that can be displaced in the direction of the center line (5) of the barrel-plunger unit (10), between an installation position (111) and an application position (112), is disposed on the blow-out pipe (54, 55) and/or on the bottom section (33),
- the adhesive disk (110) has an adhesive coating (121, 129) on each of the two face sides (113, 115), in each instance,
- the blow-out pipe (54, 55) can be sealed in the adhesive disk (110) in the installation position (111),
- the adhesive disk (110) lies against the front face side (46) of the bottom section (33), glued to it, in the application position (112), and
- the front edge (61) of the blow-out nozzle (60) lies in the plane of the front adhesive coating (121) or projects beyond this plane by at least 0.5 mm in the application position (112).

2. The barrel-plunger unit according to claim 1,
**characterized in that**
- the adhesive disk (11), when it is in the installation position (111), is at a distance of at least one millimeter from the front face side (46) of the bottom section (33), which side faces it.

3. The barrel-plunger unit according to claim 1,
**characterized in that**
- the adhesive disk (110) is an elastomer body that is configured to be elastic at least in the longitudinal direction, in other words parallel to the center line (5) of the barrel-plunger unit (10).

4. The barrel-plunger unit according to claim 1,
**characterized in that**
- the outer wall (57) of the blow-out pipe (54, 55) is shaped to be cylindrical, at least in certain regions.

5. The barrel-plunger unit according to claim 1,
**characterized in that**
- the bottom section (33) has a ring ridge (51) that extends forward as an extension of the outer cylinder wall (32).

6. The barrel-plunger unit according to claim 5,
**characterized in that**
- the ring ridge (51) has a cylindrical inner wall (52).

7. The barrel-plunger unit according to claim 1,
**characterized in that**
- the adhesive disk (110) has a bore (122) that is closed off by means of a sealing element (125).

8. The barrel-plunger unit according to claim 1,
**characterized in that**
the adhesive disk (110) consists of rubber or an elastomer or has been partially treated with silicone or teflonized.

9. The barrel-plunger unit according to claim 5,
**characterized in that**
the adhesive disk (110) has a radially projecting circumferential ridge (123) that lies against the front inner edge (59) of the ring ridge (51).

## Revendications

1. Unité cylindre-piston (10), dotée au moins d'un cylindre (20) recevant une solution d'injection, au moins d'un piston (80) et d'un revêtement adhésif (212) disposé dans la région de la face frontale libre du cylindre (20),
où
- le cylindre (20) présente un segment de base (33) sur lequel est disposé un tuyau de purge (54, 55) avec une buse de purge (60),
- au niveau du tuyau de purge (54, 55) et/ou au niveau du segment de base (33), est disposé un disque adhésif (110) élastique, pouvant être déplacé en direction de la ligne médiane (5) de l'unité cylindre-piston (10) entre une position de montage (111) et une position d'application (112),
- le disque adhésif (110) présente un revêtement adhésif (121, 129) chaque fois sur chacune des deux faces frontales (113, 115),
- dans la position de montage (111), le tuyau de purge (24, 55) peut être rendu étanche dans le disque adhésif (110),
- le disque adhésif (110) est appliqué à l'état collé dans la position d'application (112) sur la face frontale avant (46) du segment de base (33) et
- dans la position d'application (112), l'arête frontale (61) de la buse de purge (60) se situe dans le plan du revêtement adhésif (121) frontal ou dépasse de ce plan d'au moins 0,5 mm.

2. Unité cylindre-piston selon la revendication 1,
**caractérisée en ce**
- **que** le disque adhésif (110) se situant dans la position de montage (111) est éloigné de la face frontale (46) du segment de base (33) orientée vers celui-ci d'au moins un millimètre.

3. Unité cylindre-piston selon la revendication 1,
**caractérisé en ce**
- **que** le disque adhésif (110) est un corps en élastomère qui est conçu élastique au moins dans le sens longitudinal, c'est-à-dire parallèlement à la ligne médiane (5) de l'unité cylindre-piston (10).

4. Unité cylindre-piston selon la revendication 1,
**caractérisée en ce**
- **que** la paroi externe (57) du tuyau de purge (54, 55) est formée au moins partiellement de manière cylindrique.

5. Unité cylindre-piston selon la revendication 1,
**caractérisée en ce**
- **que** le segment de base (33) présente un listel (51) qui s'étend vers l'avant dans un prolongement de la paroi externe de cylindre (32).

6. Unité cylindre-piston selon la revendication 5,
**caractérisé en ce**
- **que** le listel (51) présente une paroi interne (52) cylindrique.

7. Unité cylindre-piston selon la revendication 1,
**caractérisé en ce**
- **que** le disque adhésif (110) présente un alésage (122), qui est fermé par un élément d'étanchéité (125).

8. Unité cylindre-piston selon la revendication 1,
**caractérisé en ce**
- **que** le disque adhésif (110) est constitué de caoutchouc ou d'un élastomère ou est traité partiellement avec du silicone ou est revêtu de téflon.

9. Unité cylindre-piston selon la revendication 5,
**caractérisé en ce**
- **que** le disque adhésif (110) présente une nervure circonférentielle (123) dépassant radialement, laquelle est plaquée contre l'arête interne (59) frontale du listel (51).
